# EUROPÄISCHE PATENTSCHRIFT

(11) **EP 1 511 399 B1**
(45) Veröffentlichungstag und Bekanntmachung des Hinweises auf die Patenterteilung: **29.06.2005**
(21) Anmeldenummer: 02740346.8
(22) Anmeldetag: 13.05.2002
(51) Int. Cl.: A24F 47/00, A61M 15/06

(54) **INHALATOR**
INHALER
INHALATEUR

(43) Veröffentlichungstag der Anmeldung: 09.03.2005
(73) Patentinhaber: Think Global B.V., 2011 KN Haarlem (NL); Esser, Ralf, 53773 Hennef (DE)
(72) Erfinder: ESSER, Ralf, 53773 Hennef (DE)
(74) Vertreter: Stute, Ivo, Dipl.-Ing.
(86) Internationale Anmeldenummer: PCT/DE2002/001712
(87) Internationale Veröffentlichungsnummer: WO 2003/094640

(56) Entgegenhaltungen:
- DE-A- 19 854 009
- GB-A- 2 195 899
- US-A- 5 944 025

## Beschreibung

Die Erfindung betrifft einen neuartigen Inhalator.

Inhalatoren werden meist zu medizinischen oder therapeutischen Zwecken eingesetzt und sind je nach Verwendungszweck unterschiedlich ausgestaltet.

So gibt es Inhalatoren zur Raucherentwöhnung, die aus einem Mundstück und einem Endstück bestehen und einen Luftkanal aufweisen, in den eine Nikotinkapsel eingesetzt werden kann. Durch den durch das "Ziehen" am Mundstück bewirkten Luftstrom wird in der Nikotinkapsel Nikotin freigesetzt. Solche Inhalatoren weisen den Vorteil auf, daß durch Ihre Verwendung anders als beim Rauchen von Zigaretten oder Zigarren das Raumklima und damit das Wohlempfinden Dritter nicht beeinträchtigt wird. Somit eignen sie sich insbesondere für Raucher in Nichtraucherzonen, beispielsweise in einem Flugzeug. Allerdings weist diese Art von Inhalatoren den Nachteil auf, daß das freigesetzte Nikotin weiterhin die Gesundheit des Rauchers beeinträchtigt. Auch ist das Rauchempfinden beim Inhalieren dem beim Rauchen von Zigaretten oder Zigarren kaum vergleichbar, da die eingeatmete Luft in aller Regel kalt ist und sich aufgrund des fehlenden Rauchs kein Genußgefühl einstellen wird.

Aus der DE 198 54 009 A1 ist ein zigarettenartiger Inhalator bekannt, der ebenso gegenüber herkömmlichen Zigaretten den Vorteil bieten soll, daß kein für Dritte störender Zigarettenqualm (Nebenstromrauch) entsteht. In dem vorbekannten Inhalator wird durch Erhitzen eines Substrates ein Aerosol erzeugt. Die hierfür benötigte Hitze wird durch eine flammenlose katalytische Verbrennung von Butangas, Pentan oder Isopropanol zur Verfügung gestellt. Die bei der Verbrennung entstehenden Gase werden dabei abgeführt, oder sie werden in den Strömungskanal des Inhalators eingesogen und treten zu dem inhalierten Aerosol hinzu. Bei der Verbrennung des Heizmittels läßt sich nicht vermeiden, daß für den Inhalierenden bzw. für Dritte giftige Substanzen wie CO freigesetzt werden.

Andere Inhalatoren zur medizinischen Behandlung von Atemwegserkrankungen oder Erkältungen weisen einen beheizbaren Wasserbehälter auf, auf den ein Nasen- und Mundstück aufgesetzt ist. Das Wasser im Wasserbehälter kann mit ätherischen Ölen oder pharmazeutischen Wirkstoffen versetzt werden, so daß letztere, wenn das Wasser erhitzt wird, zusammen mit dem Wasserdampf inhaliert werden können. Diese Art von Inhalatoren verwenden als Hitzequelle eine Heizwendel, die über Wechselstrom aus "der Steckdose" oder mit dem Gleichstrom beispielsweise einer Autobatterie betrieben werden kann. Sie weisen den Nachteil auf, daß sie sperrig sind. Außerdem sind sie aufgrund der benötigten Energieversorgung nicht überall verwendbar.

Diese Nachteile bestehen auch bei Inhalatoren, die aus Lösungen, die pharmazeutische Wirkstoffe enthalten, zu inhalierende Aerosole erzeugen. Für die Aerosolerzeugung werden insbesondere zwei Gerätetypen verwendet, Ultraschallvernebler und Druckvernebler. Während bei der Ultraschallverneblung die Lösung über eine durch Ultraschallwellen in Schwingung versetzte Membran vernebelt werden, strömt die Lösung bei der Druckverneblung unter Druck durch eine Düse.

Demgegenüber liegt der vorliegenden Erfindung die Aufgabe zugrunde, einen neuartigen Inhalator zu schaffen, der für die oben genannten Verwendungszwecke geeignet ist und die vorbeschriebenen Nachteile nicht aufweist.

Gelöst wird diese Aufgabe durch einen Inhalator mit den Merkmalen des Anspruchs 1.

Ein Grundgedanke der Erfindung ist es, nicht nur die bei der katalytischen Verbrennung des Wasserstoffs freiwerdende elektrische Energie, sondern auch die dabei entstehenden Abgase zur Erzeugung des mit Aromastoffen und/oder Wirkstoffen versehenen Inhalationsgemisches zu nutzen. So kann die in den Abgasen vorhandene Wärme zum Erhitzen der Inhalationszusätze genutzt werden, beispielsweise durch Erwärmung des die Inhalationszusätze beinhaltenden Behältnisses. Auch kann die Abgaswärme zum Aufheizen des Inhalationsgemisches verwendet werden, wodurch der Vorteil einer höheren Kapazität zur Aufnahme von Dampf besteht, so daß Wirkstoffe in höheren Konzentrationen als bei einem kalten Inhalationsgemisch transportiert werden können. Gleichzeitig können die Abgase direkt dem Inhalationsgemisch zugeführt werden, da sie bei der Verbrennung von Wasserstoff absolut schadstofffrei sind.

Hieraus ergibt sich eine Vielzahl von Vorteilen. So ist ein über den katalytischen Brenner mit Energie versorgter Inhalator unabhängig von extemem Energiequellen. Die benötigte Energie wird bei der katalytischen Verbrennung von Wasserstoff, umweltfreundlich freigesetzt, da das Verbrennungsprodukt lediglich unschädlicher Wasserdampf ist, der sogar zum Transport von Aroma- und Wirkstoffen verwendet wird. Der verwendete Brennstoff ist also nicht nur Energielieferant, sondern er liefert auch das Transportmittel für die Aroma- bzw. Wirkstoffe.

Grundsätzlich kann der erfindungsgemäße Katalysator konstruktiv so aufgebaut sein, daß die warmen Abgase gegebenenfalls in Verbindung mit Umgebungsluft im Betrieb des Inhalators durch das die Inhalationsstoffe enthaltende Behältnis geführt werden, wobei der Abgasstrom die Inhalationszusätze aufnimmt. Je nach Art der Inhalationszusätze/Wirkstoffe können sie dabei in flüssiger, aber auch in fester, pulvriger Form vorliegen. Liegen die Inhalationszusätze in flüssiger Form vor, können sie an ihrer Oberfläche zum Abgas verdampfen. Liegen die Inhalationszusätze in fester Form vor, können sie bei geeigneter Strömungsführung und Strömungsgeschwindigkeit der Abgase von diesen mitgerissen werden, so daß das Inhalationsgemisch ein Festkörperteilchen enthaltendes Aerosol ist.

Als katalytischer Brenner bietet sich aufgrund der einfachen kontrollierten Reaktionsführung eine Brennstoffzelle an. Moderne Brennstoffzellen weisen einen hohen Wirkungsgrad auf und können so ausgeführt sein, daß die Menge der an der katalytischen Membran der Brennstoffzelle vorbeiströmenden Luft automatisch die Menge des durch diese hindurchtretenden Wasserstoffionen steuert.

In einer vorteilhaften Ausgestaltung einer solchen als katalytischer Brenner verwendeten Brennstoffzelle ist die Brennstoffzelle spulenartig gewickelt. Aufgrund der Wicklung wird nicht nur der Raumbedarf der Brennstoffzelle erheblich reduziert, es ergibt sich darüber hinaus automatisch ein Luftkanal, durch den der Sauerstoff bzw. das diesen enthaltende Gasgemisch geführt werden kann.

Die Dosierung von in Wasser oder anderen Lösungsmitteln gelösten Inhalationszusätzen ist gegenüber der Dosierung bei pulvrigen Inhalationszusätzen einfacher und insofern bevorzugt, als keine oder nur geringe Anforderungen an die Strömungsführung und an die Strömungsgeschwindigkeit der Abgase bestehen. Je nach konstruktiver Ausgestaltung des Inhalators kann ein weiterer Vorteil darin bestehen, daß bei einer geeigneten Konzentration der gelösten Inhalationszusätze keine zusätzliche Regelung für die Zufuhr dieser Zusatzstoffe zum Inhalationsgemisch notwendig ist.

Der erfindungsgemäße Inhalator kann vorzugsweise eine mit dem katalytischen Brenner betriebene Heizeinrichtung zum Verdampfen der die Inhalationszusätze enthaltenden Lösung aufweisen, so daß die Inhalationszusätze als Dampf im Inhalationsgemisch enthalten sind. Eine solche Heizeinrichtung kann beispielsweise eine Heizwendel innerhalb des Behältnisses für die gelösten Inhalationszusätze sein.

Es ist aber auch denkbar, einen Wärmetauscher vorzusehen, über den die Wärme der Abgase an die Lösung abgegeben wird.

Auch kann der Inhalator einen vom katalytischen Brenner betriebenen Vernebler, insbesondere einen Ultraschallvernebler oder einen Druckluftvernebler zum Vernebeln der Lösung zu einem Aerosol aufweisen. Zur Verneblung der Lösung mit Druck können grundsätzlich zwei Prinzipien verwirklicht werden. Der für die Druckverneblung benötigte Druck im Behältnis, das die Lösung mit den Inhalationszusätzen enthält, kann entweder durch Erhitzen der Lösung oder aber vorzugsweise durch einen Kompressor erfolgen. Es ist beispielsweise aber auch denkbar, Lösungsmittelzusätze mit einer zu Wasser vergleichsweise niedrigen Siedetemperatur zur Druckerzeugung zu verwenden, so daß im Behältnis ein ausreichender Druck bereits bei geringer Wärmezufuhr aufgebaut werden kann. So wird es möglich, den Druck und damit die Verneblung über eine geregelte Wärmezufuhr zu steuern.

In einer weiteren vorteilhaften Ausgestaltung weist der Inhalator einen zusätzlichen Wassertank sowie eine mit dem katalytischen Brenner betriebene Heizeinrichtung zum Verdampfen des Wassers auf. In diesem Fall muß nicht das gesamte Inhalationsgemisch im katalytischen Brenner erzeugt werden. Vielmehr kann die bei der Wasserstoffverbrennung freiwerdende Wärme zur Erhitzung des Wassers zu Wasserdampf genutzt werden, der dann dem aus dem katalytischen Brenner austretenden Wasserdampf zugemischt wird.

In einer anderen bevorzugten Ausführungsform weist der Inhalator eine Regelungseinrichtung zur Dosierung der Zufuhr von Aromastoffen oder Wirkstoffen zum Inhalationsgemisch auf.

Auch ist es von Vorteil, wenn der Brennstofftank und alle anderen Behältnisse austauschbar und/oder nachfüllbar sind. So ist es möglich, den Inhalator dauerhaft zu verwenden.

Es kann im weiteren auch vorteilhaft sein, wenn in Strömungsrichtung vor dem Auslaß eine Mischvorrichtung zum Mischen des Inhalationsgemischs mit Umgebungsluft angeordnet ist. So kann die Menge des einzuatmenden Inhalationsgemisches vom Benutzer nach Bedarf dosiert werden.

Bevorzugt ist der erfindungsgemäße Inhalator mit einer Regeleinrichtung zum Regeln der durch die Brennstoffzelle geführten Sauerstoffmenge versehen. Dies bringt insbesondere dann einen Vorteil, wenn mit dem Inhalator eine zeitlich konstante Menge des Inhalationsgemisches erzeugt werden soll, wobei in diesem Fall die Funktionalität des Inhalators der eines herkömmlichen Inhalators für medizinische Zwecke entspricht.

Darüber hinaus kann ein erfindungsgemäßer Inhalator ein Mundstück aufweisen. Solch ein Mundstück kann beispielsweise in der Art eines Mundstücks für Zigaretten ausgebildet sein und ist insbesondere für Inhalatoren geeignet, bei denen das Inhalationsgemisch nur über den Mundraum aufgenommen wird. Statt eines Mundstücks kann aber auch eine Maske am Auslaß des Inhalators vorgesehen sein, mit denen Mund und Nase eines Benutzers abgedeckt werden können.

Der bei der katalytischen Verbrennung, insbesondere in einer Brennstoffzelle, entstehende Strom kann ebenso genutzt werden. So kann er einerseits zum Betrieb einer Heizwendel zum Erwärmen der Zusatzstoffe oder von Wasser genutzt werden. Er kann aber auch für eine Leuchteinheit verwendet werden. Damit kann beispielsweise der Betrieb des Inhalators angezeigt werden. Es könnte aber auch die Glut einer Zigarette oder Zigarre imitiert werden, wenn der Inhalator als Zigarettenersatz zur Rauchentwöhnung oder als neuartige Form eines Genußmittels verwendet wird.

Allen Ausführungsformen der Erfindung ist gemeinsam, daß der Inhalator ortsungebunden unabhängig von externen Stromquellen verwendet werden kann. Es kann ein für den Inhalierenden als angenehm empfundenes, warmes Inhalationsgemisch erzeugt werden, wobei auch die Verbrennungsprodukte und deren Abwärme bei Bedarf für die Erzeugung des Inhalationsgemische verwendet werden können.

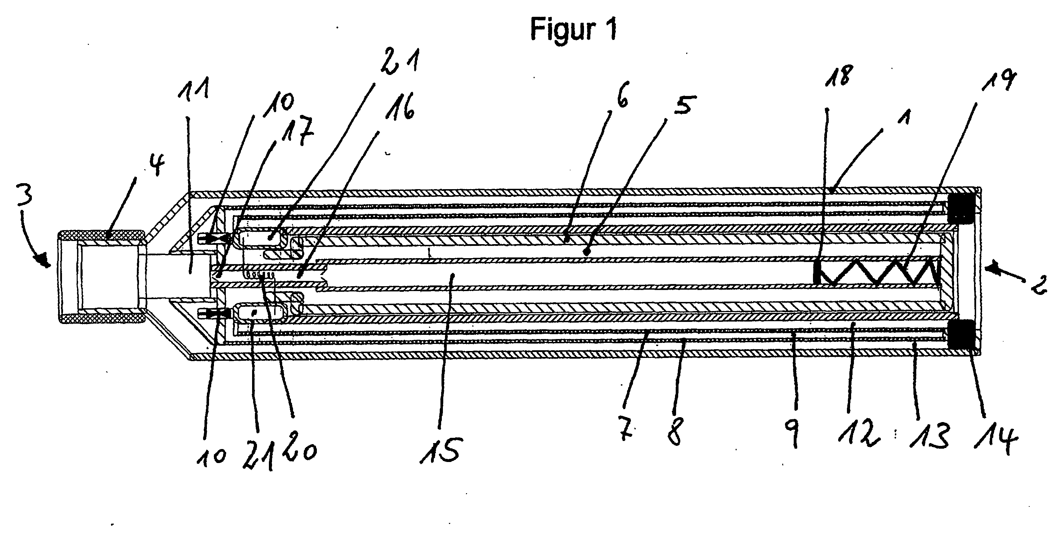

Im folgenden wird die vorliegende Erfindung anhand der das Funktionsprinzip eines Beispiels eines Inhalators darstellenden Figur **1** näher erläutert.

Der in Figur **1** im Prinzip dargestellte Inhalator weist einen länglichen, zylindrischen Hohlkörper **1** mit einem Einlaß **2** für Luft und einem Auslaß **3** für ein Inhalationsgemisch auf. Am Auslaß **3** ist ein auswechselbares Mundstück **4** vorgesehen. In seinem Inneren weist der Hohlkörper **1** eine konzentrisch angeordnete Wandung **5** auf, in der ein im wesentlichen zylindrischer, Wasserstoff enthaltender Brennstofftank **6** sitzt, dessen Boden räumlich dem Lufteinlaß **2** und dessen Gasaustritt dem Auslaß **3** zugewandt ist.

Zwischen der Wandung **5** und der Hohlkörperinnenwand und jeweils konzentrisch und beabstandet hierzu sind eine innere Membran **7** und eine äußere Membran **8,** die beide eine Brennstoffzelle bilden, angeordnet. Der zwischen den beiden Membranen **7, 8** eingeschlossene Hohlraum **9** ist auf der Seite, die dem Einlaß **2** zugewandt ist, gasdicht verschlossen. Auf seiner dem Auslaß **3** zugewandten Seite ist der Hohlraum **9** mit dem Gasaustritt am Brennstofftank **6** gekoppelt, wobei der vom Brennstofftank **6** in den Hohlraum **9** fließende Wasserstoffstrom über Ventile **10** gesteuert oder unterbrochen werden kann.

Die sich zwischen der Hohlkörperinnenwand und der äußeren Membran **8** sowie der inneren Membran **7** und der Wandung **5** ergebenden Kanäle **12, 13** sind an ihrem dem Einlaß 2 zugewandten Ende über einen ringartig ausgebildeten Luftfilter **14** mit dem Einlaß **2** verbunden. Ihr gegenüberliegendendes Ende mündet in eine in Strömungsrichtung vor dem Auslaß **3** angeordnete Mischkammer **11** (die Verbindung zwischen dem durch die innere Membran **7** und die Wandung **5** gebildeten Kanal **12** zur Mischkammer **11** ist nicht dargestellt).

Konzentrisch innerhalb des Brennstofftanks **6** ist ein zylindrischer Wirkstofftank **15** für in einer Flüssigkeit, beispielsweise in Wasser, gelöste Inhalationszusätze vorgesehen. Der Boden des Wirkstofftanks **15** ist am Boden des Brennstofftanks **6** fest verankert. Als Austritt für die Wirkstoffe ist eine Kanüle **16** vorgesehen, die durch den Gasaustritt des Brennstofftanks **6** hindurch in die Mischkammer **11** geführt ist und sich in diese hinein öffnet. Die der Mischkammer **11** zugewandte Öffnung der Kanüle **16** ist mit einem Überdruckventil **17** versehen. Innerhalb des Wirkstofftanks **15** ist ein Kolben **18** vorgesehen, der durch eine sich am Boden des Wirkstofftanks **15** abstützende Feder **19** in Richtung zur Kanüle **16** gedrückt wird. Durch diesen Druck wird die mit den Inhalationszusätzen versetzte Lösung in die Kanüle **16** gedrückt.

In der Kanüle **16** ist eine Glühwendel **20** zum Verdampfen der die Inhalationsstoffe enthaltenden Flüssigkeit angeordnet Die Glühwendel **20** kann über die Membranen **7, 8** der Brennstoffzelle mit Strom versorgt werden, ebenso wie zusätzliche Komponenten wie beispielsweise ein Kompressor für eine Druckvemebelung oder ein Ultraschallverdampfer.

Die Brennstoffzelle versorgt darüber hinaus zwei in der Wandung **5** gehaltene, wiederaufladbare Batterien **21** mit Strom. Über die Batterien **18** oder direkt über den von der Brennstoffzelle produzierten Strom kann eine hier nicht dargestellte Regelungseinrichtung zur Regelung der Wasserstoffzufuhr über die Ventile **10** vorgesehen sein. Im weiteren kann ein hier nicht dargestellter Anschluß für andere Verbraucher vorgesehen sein, der entweder direkt über die Brennstoffzelle oder über die Batterie mit Strom versorgt wird.

Zum Inhalieren setzt man den Inhalator mit seinem Mundstück **4** am Mund an und saugt über den Einlaß **2** Luft in den Inhalator ein. Dabei strömt die eingesaugte Luft an den Membranen **7, 8** vorbei und reagiert an den Membranen **7, 8** mit dem Wasserstoff, so daß ein Wasserdampf erzeugt wird und ein Luft/Wasserdampf-Gemisch entsteht.

Gleichzeitig wird durch den bei der Reaktion von der Brennstoffzelle erzeugte Strom der Glühwendel zugeführt, so daß die in der Kanüle befindliche, die Inhalationszusätze enthaltende Flüssigkeit verdampft wird. Aufgrund des hierdurch entstehenden Überdrucks öffnet sich das Überdruckventil **16,** so daß ein Inhalationszusätze enthaltender Dampf entweichen kann. Dieser Dampf wird in der Mischkammer **11** dem Luft/Wasserdampfgemisch hinzugemischt, so daß über das Mundstück ein Gemisch aus Luft, Wasserdampf und Inhalationszusätzen eingeatmet werden kann.

Damit das Inhalationsgerät dauerhaft verwendet werden kann, empfiehlt es sich, sowohl den Brennstofftank **6** als auch den Wirkstofftank **14** austauschbar mit dem Inhalator zu verbinden, oder jeweils eine Möglichkeit zum Nachfüllen vorzusehen. Hierzu kann der Hohlzylinder beispielsweise einen Ventilstutzen aufweisen, in den der Brennstofftank **6** gegebenenfalls zusammen mit dem Wirkstofftank **14** eingeschraubt werden kann.

Die Grundidee eines mit einem katalytischen Brenner betriebenen Inhalators kann in einer Vielzahl anderer konstruktiver Ausführungsformen verwirklicht werden. So ist es nicht zwingend notwendig, Membranen, Brennstoff- und Wirkstofftanks sowie Luftkanäle konzentrisch zueinander anzuordnen.

Auch können zusätzliche Steuerungs- oder Regelungsmechanismen vorgesehen sein. So sind zwar heutzutage verfügbare Brennstoffzellen in der Regel in Abhängigkeit vom zugeführten Luftstrom selbstregelnd, es könnte je nach Anwendungsbereich aber eine Regelung der Wasserstoffzufuhr notwendig sein. Auch ist es möglich, beispielsweise in Abhängigkeit von der Temperatur und/oder der relativen Luftfeuchte des Luft/Wasserdampf/Inhatationsgemisches in der Mischkammer eine regelbare Frischluftzufuhr vorzusehen, mit der das in der Mischkammer befindliche Gemisch gekühlt werden kann. Selbstverständlich kann eine gegebenenfalls notwendige Kühlung auch durch einen Wärmetauscher erfolgen.

### Bezugszeichenliste

- 1: zylindrischer Hohlkörper
- 2: Einlaß
- 3: Auslaß
- 4: Mundstück
- 5: Wandung
- 6: Brennstofftank
- 7: innere Membran
- 8: äußere Membran
- 9: Hohlraum
- 10: Ventile
- 11: Mischkammer
- 12: Kanal
- 13: Kanal
- 14: Luftfilter
- 15: Wirkstofftank
- 16: Kanüle
- 17: Überdruckventil
- 18: Kolben
- 19: Feder
- 20: Glühwendel
- 21: Batterien

## Patentansprüche

1. Inhalator mit einem katalytischen Brenner und einem Behältnis (15) für Inhalationszusätze wie Aromastoffe und/oder Wirkstoffe, mindestens einem Einlaß (2) für ein Sauerstoff enthaltendes Gasgemisch, insbesondere für Luft, und einem Auslaß (3) für ein mit Aromastoffen und/oder Wirkstoffen versehenes Inhalationsgemisch, **gekennzeichnet durch** einen mit dem katalytischen Brenner verbundenen Brennstofftank (6) für Wasserstoff.

2. Inhalator nach Anspruch 1, **dadurch gekennzeichnet, daß** der katalytische Brenner eine Brennstoffzelle (7, 8) ist.

3. Inhalator nach Anspruch 2, **dadurch gekennzeichnet, daß** die Brennstoffzelle (7, 8) spulenartig gewickelt ist.

4. Inhalator nach einem der Ansprüche 1 bis 3, **dadurch gekennzeichnet, daß** die Inhalationszusätze in Wasser oder einem anderen Lösungsmittel gelöst sind.

5. Inhalator nach Anspruch 4, **gekennzeichnet durch** eine mit dem katalytischen Brenner betriebene Heizeinrichtung (20) zum Verdampfen der die Inhalationszusätze enthaltenden Lösung.

6. Inhalator nach Anspruch 4 oder 5, **gekennzeichnet durch** einen vom katalytischen Brenner betriebenen Vernebler, insbesondere einen Ultraschallvernebler oder einen Druckluftvernebler, zum Vernebeln der Lösung.

7. Inhalator nach einem der vorstehenden Ansprüche, **gekennzeichnet durch** einen zusätzlichen Wassertank.

8. Inhalator nach einem der vorstehenden Ansprüche, **gekennzeichnet durch** eine Regelungseinrichtung zur Dosierung der Zufuhr der Inhalationszusätze zum Inhalationsgemisch.

9. Inhalator nach einem der vorstehenden Ansprüche, **dadurch gekennzeichnet, daß** der Brennstofftank (6) und alle übrigen Behältnisse (15) austauschbar und/oder nachfüllbar sind.

10. Inhalator nach einem der vorstehenden Ansprüche, **gekennzeichnet durch** eine in Strömungsrichtung vor dem Auslaß (3) angeordnete Mischvorrichtung (11) zum Zumischen von Umgebungsluft zum Inhalationsgemischs.

11. Inhalator nach einem der vorstehenden Ansprüche, **gekennzeichnet durch** eine Regeleinrichtung zum Regeln der **durch** die Brennstoffzelle geführten Sauerstoffmenge.

12. Inhalator nach einem der vorstehenden Ansprüche, **gekennzeichnet durch** ein Mundstück (4).

13. Inhalator nach einem der vorstehenden Ansprüche, **gekennzeichnet durch** eine vom katalytischen Brenner betriebene Leuchteinheit.

## Claims

1. Inhaler comprising a catalytic burner and a container (15) for inhalation additives such as aromatic substances and/or active ingredients, at least one inlet (2) for a gas mixture containing oxygen, in particular for air, and an outlet (3) for an inhalation mixture containing aromatic substances and/or active ingredients, **characterized by** a fuel container (6) for hydrogen which is connected to the catalytic burner.

2. Inhaler according to claim 1, **characterized in that** the catalytic burner is a fuel cell (7, 8).

3. Inhaler according to claim 2, **characterized in that** the fuel cell (7, 8) is wound in a coil-like fashion.

4. Inhaler according to one of claims 1 to 3, **characterized in that** the inhalation additives are dissolved in water or another solvent.

5. Inhaler according to claim 4, **characterized by** a heating device (20) actuated by the catalytic burner for the evaporation of the solution containing the inhalation additives.

6. Inhaler according to claim 4 or 5, **characterized by** a nebulizer actuated by the catalytic burner, in particular an ultrasonic nebulizer or a pneumatic nebulizer, for the nebulization of the solution.

7. Inhaler according to one of the preceding claims, **characterized by** an additional water container.

8. Inhaler according to one of the preceding claims, **characterized by** a control system for the dosing of the addition of inhalation additives to the inhalation mixture.

9. Inhaler according to one of the preceding claims, **characterized in that** the fuel container (6) and all other containers (15) are replaceable and/or refillable.

10. Inhaler according to one of the preceding claims, **characterized by** a mixing device (11) positioned upstream of the outlet (3) for the addition of ambient air to the inhalation mixture.

11. Inhaler according to one of the preceding claims, **characterized by** a control system for controlling the quantity of oxygen passed through the fuel cell.

12. Inhaler according to one of the preceding claims, **characterized by** a mouth piece (4).

13. Inhaler according to one of the preceding claims, **characterized by** a light unit actuated by the catalytic burner.

## Revendications

1. Inhalateur comprenant un brûleur catalytique, un récipient (15) pour des additifs à inhaler comme des substances aromatiques et/ou des substances actives, au moins une entrée (2) pour un mélange gazeux contenant de l'oxygène, notamment pour de l'air, et une sortie (3) pour un mélange à inhaler contenant des substances aromatiques et/ou des substances actives, **caractérisé par** un réservoir à combustible (6) pour de l'hydrogène, relié au brûleur catalytique.

2. Inhalateur selon la revendication 1, **caractérisé en ce que** le brûleur catalytique est une cellule de combustible (7, 8).

3. Inhalateur selon la revendication 2, **caractérisé en ce que** la cellule de combustible (7, 8) est enroulée à la manière d'une bobine.

4. Inhalateur selon une des revendications 1 à 3, **caractérisé en ce que** les additifs à inhaler sont dissous dans de l'eau ou dans un autre solvant.

5. Inhalateur selon la revendication 4, **caractérisé par** un dispositif de chauffage (20) actionné par le brûleur catalytique et servant à vaporiser la solution contenant les additifs à inhaler.

6. Inhalateur selon la revendication 4 ou 5, **caractérisé par** un nébuliseur actionné par le brûleur catalytique, notamment un nébuliseur à ultrasons ou un nébuliseur à air comprimé, servant à nébuliser la solution.

7. Inhalateur selon une des revendications précédentes, **caractérisé par** un réservoir à eau supplémentaire.

8. Inhalateur selon une des revendications précédentes, **caractérisé par** un dispositif de régulation pour le dosage de l'apport d'additifs à inhaler dans le mélange à inhaler.

9. Inhalateur selon une des revendications précédentes, **caractérisé en ce que** le réservoir à combustible (6) et tous les autres récipients (15) sont échangeables et/ou rechargeables.

10. Inhalateur selon une des revendications précédentes, **caractérisé par** un dispositif de mélange (11) disposé dans le sens d'écoulement avant la sortie (3) et servant à mélanger de l'air ambiant au mélange à inhaler.

11. Inhalateur selon une des revendications précédentes, **caractérisé par** un dispositif de régulation servant à réguler la quantité d'oxygène conduite dans la cellule de combustible.

12. Inhalateur selon une des revendications précédentes, **caractérisé par** un embout buccal (4).

13. Inhalateur selon une des revendications précédentes, **caractérisé par** une unité d'éclairage actionnée par le brûleur catalytique.
